# EUROPÄISCHE PATENTANMELDUNG

(11) **EP 0 782 999 A1**
(43) Veröffentlichungstag der Anmeldung: **09.07.1997**
(21) Anmeldenummer: 96120952.5
(22) Anmeldetag: 27.12.1996
(51) Int. Cl.: C07F 13/00, C11D 3/39, B01J 31/18, C07D 303/04

(54) **Verfahren zur Oxidation organischer Verbindungen in Gegenwart von Bis- und Tris-(mu-oxo)-di-Mangan-Komplexsalzen als Katalysator**

(30) Priorität: 04.01.1996 DE 19600160
(71) Anmelder: HOECHST AKTIENGESELLSCHAFT, 65929 Frankfurt am Main (DE)
(72) Erfinder: Tafesh, Ahmed, Dr., 78413 Corpus Christi, Texas (US); Beller, Matthias, Prof. Dr., 85747 Garching (DE)

(57) **Zusammenfassung**

Verwendung von Bis- und Tris-(µ-oxo)-di-Mangan-Komplexsalzen der Formel

[LMn(µ-O)ₐ(µ-OAc)_{b}MnL]^{x}A_{y}

als Katalysator zur Oxidation organischer Verbindungen, wobei in obiger Formel
- Ac: eine C₂-C₈-Acylgruppe ist,
- a: ist 1, 2 oder 3,
- b: ist 0, wenn a 2 oder 3 ist oder b ist 2, wenn a 1 ist,
- x: bezeichnet die Anzahl der positiven Ladungen und ist 2 oder 3,
- A: ist ein Anion,
- y: bedeutet die für den Ausgleich der positiven Ladungen erforderliche equivalente Menge an Anion A und
- L: bedeutet einen Ligand, vorzugsweise [N,N-bis(2-pyridyl(methyl-)N-methylamin oder [N,N,N',N'-tetrakis-(2-pyridylmethyl-)1,2-ethylendiamin.

## Beschreibung

Es sind bereits eine Reihe von Bis- und Tris-(µ-oxo-di-Mangan-Komplexsalzen vorbeschrieben, so beispielsweise in WO 93/25562, Inorg. Chem. 1989, 28, p. 3606-3608; J. Chem. Soc. Chem. Comm. 1995, p. 949-950; Inorg. Chem. 1994, 33, 4105-4111 und Nature, Vol. 369, p. 637-639.Mangan-Komplexsalze dieser Art sind außerdem in EP-A-0 458 397; EP-A-0 458 398 und EP-A-0 544 519 beschrieben. Diese Mangan-Komplexsalze, die als Katalysatoren für oxidative Bleichmittel dienen, enthalten einen Liganden vom Triazacyclononan-Typ, der jedoch nur in einem mehrstufigen Prozeß hergestellt werden kann und somit schwer zugänglich ist. Es wurden nun überraschenderweise weitere, bisher nicht beschriebene, Mangan-Komplexsalze dieser Art gefunden, die sich ebenfalls sehr gut als Katalysatoren für Oxidationen eignen, die aber leichter zugängliche Liganden enthalten, was einen bedeutenden technischen Fortschritt darstellt.

Gegenstand der Erfindung ist ein Verfahren zur Oxidation organischer Verbindungen, vorzugsweise einer organischen Verbindung, die mindestens eine elektronenreiche Verbindung aufweist, beispielsweise zumindest eine Doppel- oder Dreifach-C-C-Bindung, z.B. Vinyl- oder Styrol-Verbindung, wobei man die Oxidation in Gegenwart von Bis- und/oder Tris-(µ-oxo)-di-Mangan-Komplexsalzen der Formel I

[LMn(µ-O)ₐ(µ-OAc)_{b}MnL]^{x}A_{y} (I)

durchführt, worin
- Ac: eine C₂-C₈-Acylgruppe ist,
- a: ist 1, 2 oder 3,
- b: ist 0, wenn a 2 oder 3 ist oder b ist 2, wenn a 1 ist,
- x: bezeichnet die Anzahl der positiven Ladungen und ist 2 oder 3,
- A: ist ein ein- oder zweifach negativ geladenes Anion,
- y: bedeutet die für den Ausgleich der positiven Ladungen erforderliche equivalente Menge an Anion A und
- L: bedeutet einen Liganden der Formel II oder III 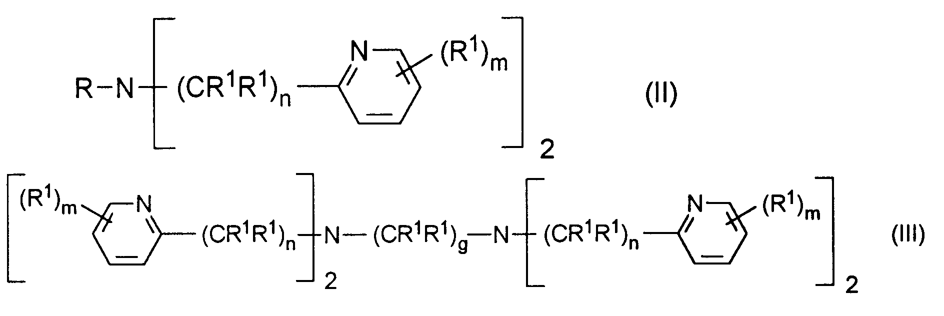
worin R C₁-C₁₂-Alkyl, C₅-C₁₀-Cycloalkyl, Phenyl, NH₂, NHR², N(R²)₂, OH, OR² oder COOH, R¹ Wasserstoff, C₁-C₁₂-Alkyl, C₅-C₁₀-Cycloalkyl, NH₂, NHR², N(R²)₂, OH, OR², COOH, COOR², Cl, Br, F, J oder CN, R² C₁-C₁₂-Alkyl oder C₅-C₁₀-Cycloalkyl bedeuten, g 2 oder 3 und m und n Null oder eine ganze Zahl von 1 bis 4 bedeuten.

Bevorzugt sind solche Liganden der Formeln I und II, bei denen alle Substituenten R¹ Wasserstoff bedeuten und n = 1 ist. Für den Fall a = 3 und b = 0 ist L vorzugsweise ein Ligand der Formel II. Bevorzugt sind auch Komplexsalze der Formel I, worin a = 1 und b = 2 ist und L einen Liganden der Formel III bedeutet. Bei Verbindungen der Formel I, worin L einen Liganden der Formel II bedeutet, ist R vorzugsweise C₁-C₄-Alkyl, insbesondere Methyl. Als Anion A kommen in Frage F⁻, Cl⁻, Br⁻, J⁻, NO₃⁻, ClO₄⁻, NCS⁻, PF₆⁻, RSO₃⁻, RSO₄⁻, SO₄²⁻, BPh₄⁻ sowie die Anionen von aliphatischen und aromatischen Alkoholen sowie aliphatischen und aromatischen Carbonsäuren (Streitweiser & Heathcock, Introduction to Organic Chemistry, 1976, Chapter 17, App.IV). Bevorzugte Anionen sind PF₆⁻, ClO₄⁻, Tosylat, Acetat und Benzoat.

Die Herstellung der in den erfindungsgemäßen Mangan-Komplexslazen enthaltenen Liganden der Formel III erfolgt beispielsweise durch Umsetzung von 2-(Chlormethyl)pyridiniumchlorid mit Ethylendiamin in Gegenwart eines Phasentransferkatalysators (s. Synthesis, June 1992, p. 539-540) bzw. in analoger Weise für die substituierten anderen Liganden der Formel III. In gleicher Weise werden die Liganden der Formel II hergestellt durch Umsetzung von 2-(Chlormethyl)pyridiniumchlorid bzw. von analogen Pyridiniumverbindungen mit einem Amin R-NH₂. Die Herstellung der erfindungsgemäßen Mangan-Komplexsalze der Formel I erfolgt entsprechend den Angaben in Inorg. Chem. 1989, 28, 3606-3608, indem man eine wäßrige Lösung, die MnCl₂ und den Liganden enthält, mit H₂O₂ oxidiert oder analog zu dem in Inorg. Chem. 1994, 33, 4105-4111 beschriebenen Verfahren, bei dem die Oxidation mit Ammoniumperoxodisulfat vorgenommen wird.

Das Verfahren zur Herstellung eines Salzes der Formel I kann das Oxidieren einer Lösung beinhalten, die Mn²⁺ und L enthält. Das Oxidieren kann mit einem Peroxid oder Peroxidsulfat erfolgen, wobei das Peroxid H₂O₂ sein kann und das Peroxidsulfat Ammoniumperoxidsulfat. Die Lösung kann eine wässrige Lösung sein und das Mn²⁺ kann in der Form von MnAy, wie z.B. MnCl₂, vorliegen.

Die so erhaltenen Mangan-Komplexsalze der Formel I werden erfindungsgemäß als Katalysatoren für die Oxidaton von organischen Verbindungen eingesetzt, wobei als organische Verbindugen bei der Oxidation alle organischen Verbindungen eingesetzt werden können, jedoch sind Verbindungen die mindestens eine elektronenreiche Bindung aufweisen, wie z.B. eine Doppel- oder Dreifachbindung, bevorzugt. Ein Beispiel für eine ungesättigte Bindung ist ein Alken oder ein Alkin.

Besonders bevorzugt ist die Verwendung des Salzes der Formel I für die Oxidation einer Vinylverbindung der Formel R-CH = CH₂, in der R ein aromatischer Rest, vorzugsweise Phenyl, oder ein C₁-C₁₀-Alkyl-Rest ist, der unsubstituiert sein kann oder substituiert, beispielsweise durch ein F, Cl, J, Br, OH, OR², CN, NH₂, NHR², N(R²), wobei R² wie oben definiert ist. Bei der organischen Verbindung die auf diese Weise in Gegenwart eines Salzes der Formel I oxidiert werden kann, kann es sich um Styrol oder um substituierte Styrole handeln.

### Herstellungsbeispiele

### Beispiel 1

### Tris-(µ-oxo)bis[N,N-bis(2-pyridylmethyl-)N-methylamin]dimangan IV, IV hexafluorophosphat

1,74 g (8,8 mmol) MnCl₂·4H₂O, 1,88 g (8,8 mmol) N,N-bis-(2-pyridylmethyl)-N-methylamin und 1,75 g (9,5 mmol) KPF₆ wurden in 600 ml einer Mischung aus Ethanol und Wasser (2:1) gelöst. Die Lösung wurde 20 Minuten bei Raumtemperatur gerührt und dann im Eisbad auf 5°C gekühlt. Dann wurden 10 ml H₂O₂ (3 %ig) und 2,6 ml einer 20 %igen wäßrigen NaOH-Lösung zugegeben, wobei die Temperatur auf 10°C anstieg und ein Niederschlag ausfiel. Diese Mischung wurde 1 Stunde lang bei 5°C und eine weitere Stunde bei 20°C gerührt. Der Niederschlag wurde abfiltriert und mit Wasser gewaschen. Rohausbeute 6,1 g. Nach dem Trocknen wurde der Feststoff mit Aceton behandelt, wobei MnO₂ als Feststoff zurückblieb. Die Acetonlösung wurde vom unlöslichen MnO₂ abfiltriert und das Aceton abdestilliert. Man erhielt 1,25 g eines grau-grünen Feststoffs.

| | | | | |
|---|---|---|---|---|
| Analyse: | ber.: | C 40,9 % | H 3,8 % | N 10,9 % |
| | gef.: | C 39,5 % | H 3,6 % | N 10,4 % |

### Beispiel 2

### Tris-(µ-oxo)bis[N,N,N',N'-tetrakis(2-pyridylmethyl-)1,2-ethylendiamin]dimangan IV,IV-hexafluorophosphat

Eine Mischung aus 25,5 ml Ethanol und 4,5 ml Wasser wurde durch dreimaliges Anlegen eines Vakuums entgast und mit Argon behandelt, um alle Reste an Sauerstoff zu entfernen, die sonst eine Oxidation von MnII zu MnIV bewirken würde. Zu dieser Mischung wurden 2,47 g (5,82 mmol) N,N,N',N'-Tetrakis(2-pyridylmethyl)-ethylendiamin gegeben. Man erhielt eine gelbe Lösung mit einem pH-Wert von 8,8. Dann wurden 0,96 g (3,6 mmol) Mangantriacetat (Mn(OAc)₃·3H₂O) zugegeben. Die Lösung wurde braun und wies einen pH-Wert von 6,2 auf. Anschließend wurden 2 g (14,58 mmol) Natriumacetat zugegeben (pH 6,5), dann Perchlorsäure (55 Tropfen) zu einem pH-Wert von 5. Nach Zugabe von 3 g (24,48 mmol) Natriumperchlorat fiel bei pH 6,1 ein Niederschlag aus. Die Reaktionsmischung wurde 4 Stunden lang gewährt. Die ausgefallenen Kristalle wurden abgefiltert und unter Stickstoff gefiltert. Man erhielt 3,08 g Rohprodukt der Verbindung (µ-Oxo) bis (µ-acetatobis[N,N,N',N'-(2-pyridylmethyl-)ethylendiamin-]di-mangan-III;III-perchlorat.

1,15 g (0,823 mmol) dieser Verbindung wurden in 40 ml einer 1:1-Mischung aus Ethanol und Wasser gelöst. Nach einer halben Stunde wurden 4 ml Triethylamin zugegeben. Der pH-Wert der Reaktionsmischung betrug 11. Dann wurden 3,89 g (21,12 mmol) KPF₆ zugegeben. Nach 15 Minuten wurde die Reaktionsmischung filtriert, um unlösliches Mangandioxid abzutrennen. Aus dem Filtrat wurden die Lösemittel abdestilliert und der Rückstand mit Aceton behandelt, um auf diese Weise Restmengen an Mangandioxid abzutrennen, denn das gewünschte Komplexsalz ist im Gegensatz zu Mangandioxid gut in Aceton löslich. Nach Abtrennen des Mangandioxids wurde das Aceton destillativ abgetrennt. Man erhielt die Titelverbindung in Form eines weißen Pulvers.

### Beispiel 3

### (µ-Oxo)bis(µ-butyrato)bis[N,N,N',N'-tetrakis(2-pyridylmethyl-)1,2-ethylendiamin]dimangan III,IV perchlorat

2,47g (5,82 mmol) N,N,N',N'-Tetrakis-(2-pyridylmethyl)-1,2-ethylendiamin wurden in 30 ml einer Mischung aus Ethanol und Wasser (5:1) gelöst und anschließend Mn(O Acetat)₃·2H₂O (0,96 g; 3,6 mmol) hinzugegeben. Dann wurde 2,2 g (20 mmol) Natriumbutyrat zugegeben und anschließend 2 ml HClO₄ (70 %ig). Es stellte sich ein pH-Wert von 7,5 ein. Dann wurden 3 g (24,48 mmol) NaClO₄ zugegeben und diese Mischung wurde 4 Stunden bei Raumtemperatur gerührt. Es bildete sich ein Niederschlag, der abfiltriert, mit einer Ethanol/Wasser-Mischung (85 % EtOH/15 % H₂O) gewaschen und getrocknet wurde. Rohausbeute 2,3 g. Der Feststoff wurde mit 70 ml Aceton behandelt und die Aceton-Lösung von den unlöslichen anorganischen Salzen abfiltriert. Nach Abdestillation des Acetons und Umkristallisation mit Ethanol/Wasser 5:1 erhielt man das oben genannte Komplexsalz.

| | | | | | |
|---|---|---|---|---|---|
| Analyse: | ber.: | Mn 7,5 % | C 49,17 % | H 4,95 % | N 11,47 % |
| | gef.: | Mn 8,0 % | C 44,8 % | H 4,5 % | N 12,1 % |

### Beispiel 4

### Bis(µ-oxo)bis[N,N,N',N'-tetrakis(2-pyridylmethyl-)1,2-ethylendiamin]dimangan III,IV perchlorat

0,425 g (1 mmol) N,N,N',N'-tetrakis-(2-pyridylmethyl)-1,2-ethylendiamin und 0,198 g (1 mmol) MnCl₂·4H₂O wurden in 6 ml Wasser gelöst. Die Lösung wurde 30 Minuten bei Raumtemperatur gerührt. Dann wurden 4 Tropfen H₂O₂ zugegeben und eine Stunde bei Raumtemperatur gerührt. Dann wurden 0,13 g (1 mmol) NaClO₄ zugegeben und 3,5 Stunden bei Raumtemperatur gerührt. Das ausgefallene Mangan-Komplexsalz wurde abfiltriert und mit einer 1-molaren NaClO₄-Lösung gewaschen.

| | | | | | | |
|---|---|---|---|---|---|---|
| Analyse: | ber.: | Mn 8,4 % | C 47,77 % | H 4,47 % | Cl 8,14 % | N 12,86 % |
| | gef.: | Mn 8,4 % | C 45,1 % | H 4,3 % | Cl 8,6 % | N 11,9 % |

Der in den vorherigen Beispielen benutzte Ligand N,N,N',N'-Tetrakis-(2-pyridylmethyl)ethylendiamin wurde entsprechend den Angaben in Synthesis, 1992, S. 539 hergestellt.

### Beispiel 5

### Bis(µ-oxo)bis[N,N,N',N'-tetrakis(2-pyridylmethyl-)1,2-ethylendiamin]di-mangan III,IV peroxodisulfat

Eine Mischung aus 4,6 g (10,5 mmol) N,N,N',N'-tetrakis-(2-pyridylmethyl-)1,2-ethylendiamin und 1,98 g MnCl₂-4H₂O wurde eine Stunde lang in 15 ml (1,5 mmol) 0,1 M HCl gerührt. Dann wurde das Wasser abdestilliert und eine Lösung von 7,5 g (32,8 mmol) (NH₄)₂ S₂O₈ in 20 ml Wasser zugegeben. Diese Mischung wurde eine Stunde bei Raumtemperatur gerührt. Es bildete sich ein Niederschlag, der abfiltriert und mit Wasser gewaschen wurde. Nach Trocknen unter Vakuum erhielt man 7,9 g des Rohprodukts. Das Rohprodukt wurde 30 Minuten lang mit 100 ml Aceton behandelt. Das dabei als Feststoff verbliebene Mangandioxid wurde abfitriert und die Aceton-Lösung wurde im Vakuum eingeengt. Nach Trocknen im Vakuum erhielt man 6,30 g des oben genannten Mangan-Komplexsalzes.

| | | | | | |
|---|---|---|---|---|---|
| Analyse: | ber.: | Mn 8,47 %; | C 48, 15 %; | H 4,5 %; | N 12,96 % |
| | gef.: | Mn 8,1 %; | C 43,9 %; | H 3,7 %; | N 11,9 %. |

### Anwendungsbeispiele

### Beispiel 1

### Oxidation von Styrol

Zu einer Mischung aus 0,208 g (2 mmol) Styrol in 20 ml CH₂Cl₂ und 1,68 g (20 mmol) Natriumhydrogencarbonat in 20ml Wasser wurden 0,0178 g (0,02 mmol) des Komplexsalzes gemäß Herstellbeispiel 1 gegeben. Nach einer Reaktionszeit bei Raumtemperatur von 10 Minuten wurden 20 ml H₂O₂ (30 %ig) langsam über 2,5 Stunden zugegeben wobei die Temperatur auf 28°C anstieg. Nach weiteren 22 Stunden war die Reaktion beendet. Die Schichten wurden getrennt und die organische Phase wurde unter Vakuum eingeengt. Das erhaltene Produkt wurde auf einer Säule chromatographisch gereinigt und man erhielt Styroloxid in einer Ausbeute von 54 % bezogen auf Styrol.

### Beispiel 2

### Oxidation von 3-Chlorstyrol

2 mmol 3-Chlorstyrol in 20 ml CH₂Cl₂ wurde mit 0,0178 g (0,02 mmol) des Komplexsalzes gemäß Herstellbeispiel 1 in Gegenwart von 1,68 g (20 mmol) Natriumhydrogencarbonat analog zu dem Verfahren gemäß Anwendungsbeispiel 1 oxydiert. Man erhielt 3-Chlor-styroloxid in einer Ausbeute von 49 % bezogen auf 3-Chlor-styrol.

### Beispiel 3

### Herstellung von 1-Chlor-2-phenylethanol aus Styrol

Zu einer Mischung aus 0,208 g (2 mmol) Styrol in 20 ml CH₂Cl₂ und 1,68 g (20 mmol) Natriumhydrogencarbonat in 20 ml Wasser wurden 0,0178 g (0,02 mmol) des Komplexsalzes gemäß Herstellbeispiel 1 gegeben. Nach einer Reaktionszeit bei Raumtemperatur während 10 Minuten wurden innerhalb von 15 Minuten 1,5 g (20 mmol) NaCl langsam zugegeben, wobei die Temperatur auf 28°C anstieg. Nach weiteren 6 Stunden war die Reaktion beendet. Die Schichten wurden getrennt und die organische Phase wurde im Vakuum eingeengt. Das erhaltene Produkt wurde auf einer Säule chromatographisch gereinigt. Man erhielt 1-Chlor-2-phenylethanol in einer Ausbeute von 40 % bezogen auf Styrol.

### Beispiel 4

Eine Mischung aus 0,208 g Styrol (2 mmol) in 10 ml CH₂Cl₂ und 1,68 g (20 mmol) Natriumhydrogencarbonat in 20 ml Wasser und zusammen mit 0,026 g (0,02 mmol) des Komplexsalzes gemäß Herstellbeispiel 5 10 Minuten lang bei Raumtemperatur gerührt. Dann wurden langsam über einen Zeitraum von 2,5 Stunden 20 ml (200 mmol) H₂O₂ (30 %ig) zugegeben, wobei die Temperatur auf 28°C stieg. Die Reaktionsmischung wurde weitere 22 Stunden gerührt und dann die organische Phase von der wäßrigen Phase getrennt. Die organische Phase wurde im Vakuum eingeengt und der Rückstand wurde chromatographisch über eine Säule gereinigt. Man erhielt Styroloxid in einer Ausbeute von 49,6 %.

### Beispiel 5

Eine Mischung aus 0,208 g Styrol in 2 ml Methanol und 40 ml einer wäßrigen Lösung von Natriumcarbonat wurden zusammen mit 0,26 g (0,2 mmol) des Komplexsalzes gemäß Herstellbeispiel 5 10 Minuten lang bei Raumtemperatur gerührt. Die Menge an Natriumcarbonat war so bemessen, daß der pH-Wert des Reaktionsansatzes bei 6,5 lag. Dann wurden langsam über einen Zeitraum von 5 Stunden 20 ml (200 mmol) H₂O₂ (30 %ig) zugegeben, wobei die Temperatur auf 28°C stieg. Die Reaktionsmischung wurde weitere 24 Stunden gerührt und dann die wäßrige Phase von der organischen Phase getrennt. Die organische Phase wurde im Vakuum eingeengt und der Rückstand wurde chromatographisch über eine Säule gereinigt. Man erhielt Benzaldehyd in einer Ausbeute von 48 %.

## Patentansprüche

1. Verfahren zur Oxidation organischer Verbindungen, dadurch gekennzeichnet, daß man die Oxidation in Gegenwart von Bis- und Tris-(µ-oxo)-di-Mangan-Komplexsalzen der Formel I
[LMn(µ-O)ₐ(µ-OAc)_{b}MnL]^{x}A_{y} (I)
durchführt, worin
Ac eine C₂-C₈-Acylgruppe ist,
a ist 1, 2 oder 3,
b ist 0, wenn a 2 oder 3 ist oder b ist 2, wenn a 1 ist,
x bezeichnet die Anzahl der positiven Ladungen und ist 2 oder 3,
A ist ein ein- oder zweifach negativ geladenes Anion,
y bedeutet die für den Ausgleich der positiven Ladungen erforderliche equivalente Menge an Anion A und
L bedeutet einen Liganden der Formel II oder III 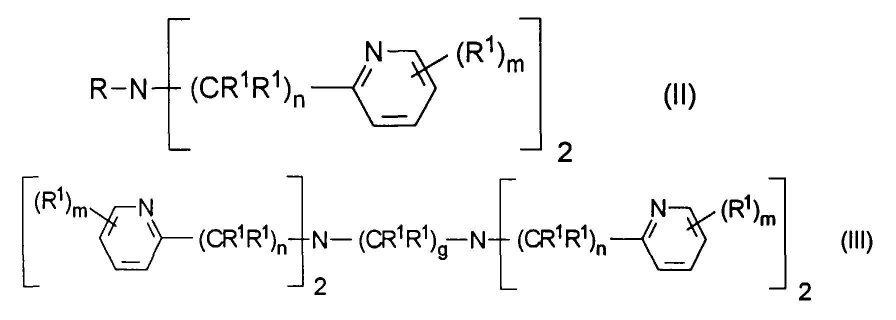
worin R C₁-C₁₂-Alkyl, C₅-C₁₀-Cycloalkyl, Phenyl, NH₂, NHR², N(R²)₂, OH, OR² oder COOH, R¹ Wasserstoff, C₁-C₁₂-Alkyl, C₅-C₁₀-Cycloalkyl, NH₂, NHR², N(R²)₂, OH, OR², COOH, COOR², Cl, Br, F, J oder CN, R² C₁-C₁₂-Alkyl oder C₅-C₁₀-Cycloalkyl bedeuten, g 2 oder 3 und m und n Null oder eine ganze Zahl von 1 bis 4 bedeuten.

2. Verfahren nach Anspruch 1, dadurch gekennzeichnet, daß man die Oxidation in Gegenwart von Mangan-Komplexsalzen der Formel I durchführt, worin L einen Liganden der Formel II oder III darstellt, worin R¹ Wasserstoff bedeutet.

3. Verfahren nach Anspruch 1, dadurch gekennzeichnet, daß man die Oxidation in Gegenwart von Mangan-Komplexsalzen der Formel I durchführt, worin L einen Liganden der Formel II darstellt, worin R C₁-C₄-Alkyl bedeutet.

4. Verfahren nach Anspruch 1, dadurch gekennzeichnet, daß man Vinylverbindungen der Formel
R-CH=CH₂
oxidiert, worin R einen aromatischen Rest oder C₁-C₁₀-Alkyl bedeutet.

5. Verfahren nach Anspruch 1, dadurch gekennzeichnet, daß man Styrol oder substituierte Styrole oxidiert.
